(19) **Europäisches Patentamt / European Patent Office / Office européen des brevets**

(11) **EP 3 416 697 B1**

(12) **EUROPEAN PATENT SPECIFICATION**

(45) Date of publication and mention of the grant of the patent:
**20.07.2022 Bulletin 2022/29**

(21) Application number: **17703171.3**

(22) Date of filing: **09.02.2017**

(51) International Patent Classification (IPC):
**A61M 1/06** (2006.01)

(52) Cooperative Patent Classification (CPC):
**A61M 1/06;** A61M 2205/10; A61M 2205/42;
A61M 2209/08

(86) International application number:
**PCT/EP2017/052796**

(87) International publication number:
**WO 2017/140562 (24.08.2017 Gazette 2017/34)**

(54) **DRIVE UNIT FOR A BREAST PUMP AND BREAST PUMP**

ANTRIEBSEINHEIT FÜR EINE BRUSTPUMPE UND BRUSTPUMPE

UNITÉ D'ENTRAÎNEMENT POUR TIRE-LAIT ET TIRE-LAIT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.02.2016 EP 16156587**

(43) Date of publication of application:
**26.12.2018 Bulletin 2018/52**

(60) Divisional application:
**22163489.2**

(73) Proprietor: **Koninklijke Philips N.V.**
**5656 AG Eindhoven (NL)**

(72) Inventors:
• **KEMPS, Peter Adrianus Albert**
**5656 AE Eindhoven (NL)**
• **VAN ROOIJEN, Alexander**
**5656 AE Eindhoven (NL)**
• **GEERLINGS, Alexander Cornelis**
**5656 AE Eindhoven (NL)**

(74) Representative: **Roche, Denis**
**Société Civile SPID**
**33, rue de Verdun**
**92150 Suresnes (FR)**

(56) References cited:
EP-A1- 2 105 151        EP-A1- 2 196 229
EP-B1- 2 105 151        WO-A1-2004/112873
CN-U- 203 763 554       US-A1- 2001 038 799
US-A1- 2012 116 299

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

The present invention relates to a drive unit for a breast pump and to a breast pump for expressing breast milk from a female breast.

BACKGROUND OF THE INVENTION

Noise- and vibration-reducing arrangements are known from various fields of application, since most motor-driven devices will emit more or less noise and might vibrate thus impeding use and handling of the devices.

US 2005/0168046 A1 discloses a method and an apparatus for damping vibrations in a vehicle seat. Selected components of the seat are decoupled from the other components by elastomeric elements and tuned to resonant frequencies to absorb noise and vibrations. Each of the components can be tuned to the required resonant frequencies to cooperate and act as a single mass damper. However, the proposed solution of US 2005/0168046 A1 needs components whose vibration frequency fits in the range of frequencies to be eliminated. For many applications, there won't be enough components or their frequency won't fit to effectively eliminate noise and vibrations.

EP 2 196 229 A1 describes a motorised head for a breast pump comprising a motor unit including a motor and a drive mechanism for driving a pump, a body mounting portion which is fixedly mountable to a body of a breast pump, a motor mounting portion on which the motor and drive mechanism are disposed, and a resiliently deformable member connecting the body mounting portion and the motor mounting portion such that the resiliently deformable member reduces the transfer of vibration caused by the motor or drive mechanism to a breast pump body to which the body mounting portion is mounted.

EP 2 105 151 A1 refers to a suction pump comprising a ventilating valve having a ventilating body which is operated in such a way that it releases only a partial region of a ventilating opening when the valve is open and subsequently releases a larger region or the whole ventilating opening. The ventilating body is a ventilating membrane having an edge region. The ventilating membrane is connected to an anchor of a lifting magnet via a connecting pin. The connecting pin is arranged over one corner of the ventilating opening.

CN 203763554 U describes an electric breast pump with a housing and a motor part. The housing has two parts being connected to each other by way of clips comprising a resilient material to avoid transfer of vibrations.

US 2001/038799 A1 refers to a motorized pump that includes a flexible diaphragm fitting within a rigid member, a motor drive mechanism for drawing a puller member attached to the diaphragm away from the rigid member to create a space between the diaphragm and the rigid member and form a negative pressure region within that space, and an outlet communicating with the negative pressure region. A thermoplastic elastomere molded into the housing is used for dampening.

US 2012/0116299 A1 describes a drive mechanism for generating a negative pressure in a vacuum chamber of a breast pump, the drive mechanism comprising a housing, a motor coupled to a rotatable drive element within the housing, a resilient membrane coupled to the housing and, a flexible strap coupled between the drive element and the resilient membrane. Rotation of the drive element causes the flexible strap to pull and resiliently deform the resilient membrane to create the negative pressure in a vacuum chamber of a breast pump.

SUMMARY OF THE INVENTION

It is an object of the present invention to provide a breast pump which is effectively reduced in noise in the most bothersome frequency ranges, i.e. in high and mid frequencies, by simple and cost-effective features which do not require additional components or complex calculation models.

The invention is defined by the features of independent claim 1.

In a first aspect of the present invention a drive unit for a breast pump is presented that comprises a housing, internal components arranged in the housing, and a suspension, wherein the internal components are mechanically coupled to each other to form a unit which is mechanically coupled to the housing by way of the suspension. The suspension comprises at least one resilient element mechanically coupling the unit of components to the housing. The resilient element is a spring, preferably a coil spring or a plate spring. All internal components are mechanically coupled. Thus the complete mass of the components can be used to reduce noise and vibration emissions. The internal components are fixedly or removably arranged on a base structure, the base structure being mechanically coupled to the housing by way of the suspension. All internal components arranged in the housing are mechanically coupled and are arranged on the suspension via the base structure.

On the one hand, the base structure contributes to the total mass of the components, thus increasing the effect of noise reduction, on the other hand the base structure allows easy mounting during production since the components can be pre-assembled before inserted into the housing.

In a second aspect of the present invention a breast pump is presented comprising a drive unit and an expression kit for positioning on a user's breast to express breast milk therefrom.

Due to the dependency of the damping behavior of a device on the mass of the components involved, an increase of the mass by combining the components will lead to a reduction in noise and vibrations in the discomforting high and mid-range frequency regions. Springs like coil springs and plate springs are cheap and easy to fit parts which can be used in nearly any configuration to obtain the result of optimized dampening and noise reduction. The remaining frequencies are less disturbing and thus the use of the breast pump is more comfortable for the user. The springs can also be used to backfit used devices to improve their performance. As a consequence, the drive unit and thus the breast pump are more comfortable for the user and the environment. Use of the breast pump can then be more effective due to the comfortable handling and the relaxation of the user. The user will use the breast pump more frequently and to a more effective result e.g. in terms of milk volume expressed.

Preferred embodiments of the invention are defined in the dependent claims. It shall be understood that the claimed method has similar and/or identical preferred embodiments as the claimed device and as defined in the dependent claims.

The internal components may comprise at least a pressure unit, the pressure unit comprising a pump and a motor for driving the pump, a control unit and a user interface. The pressure unit may be arranged to generate a positive pressure or to generate a negative pressure. The latter is also known as a vacuum unit. Any other component present in the housing, like stabilizing elements, control elements etc. can be used to contribute to the resulting mass and thus increase the effect of noise reduction.

The resilient element may further include a damping element or an elastomeric element. The choice of the resilient element can be optimized according to the respective application and conditions like the available space.

According to a preferred embodiment, the damping element is adjustable. This can help further eliminating certain frequencies.

Preferably two or more resilient elements are provided, the resilient elements being of the same or of different types. This provides the possibility to combine the damping characteristics of different shapes and materials.

According to an advantageous embodiment of the invention, the two or more resilient elements are arranged to stabilize the internal components in the housing. This allows protecting the internal components from damage during handling by tilts, shocks and drops.

The elastomeric element preferably comprises silicone, polyurethane, natural or synthetic rubber, an elastomeric injection molded polymer or a combination thereof. Such materials are easy to process and allow designing the elastomeric elements according to the demands.

Alternatively, the internal components can be fixedly or removably coupled to each other. This is a cost-effective possibility which also does not require additional parts.

In an advantageous embodiment of the breast pump the expression kit is fixedly or removably connected to the pressure unit. This provides an easy and compact assembly without additional components.

Preferably, the expression kit is fixedly or removably connected to the housing of the drive unit by way of a resilient flange with the flange being connected to the pressure unit. Thus a transfer of noise or vibrations to the expression kit can be avoided.

BRIEF DESCRIPTION OF THE DRAWINGS

These and other aspects of the invention will be apparent from and elucidated with reference to the embodiment(s) described hereinafter. In the following drawings

    Fig. 1 shows a schematic view of a breast pump with a drive unit suitable for implementation of the invention,
    Fig. 2A shows a simplified schematic view of a drive unit of a breast pump according to the state of the art, and
    Fig. 2B shows a simplified schematic view of a drive unit of a breast pump according to the invention.

DETAILED DESCRIPTION OF THE EMBODIMENTS

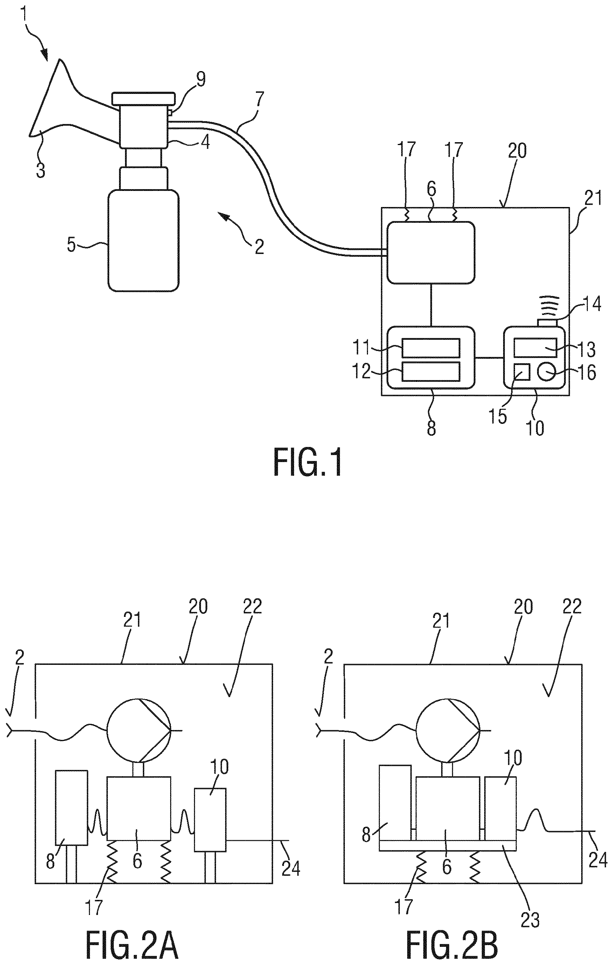

Fig. 1 shows a schematic view of a breast pump 1 which is suitable for implementation of the inventive features. The breast pump 1 comprises an expression kit 2 with an expression kit housing 4. A funnel 3, a container 5 and a pipe 7 are connected to the expression kit housing 4. The funnel 3 is shaped to be placed on a user's breast to express breast milk therefrom. The breast milk is collected in the container 5 which can be connected to the expression kit housing 4 for example by screwing or by a bayonet fixing. A venting valve 9 can be provided in the expression kit housing 4 for venting the components during and after expression. The expression kit 2 is connected to a drive unit 20 via the pipe 7.

The drive unit 20 comprises a housing 21 which contains all internal components 22 necessary for operation of the breast pump 1. The components 22 comprise at least a pressure unit 6, a control unit 8 and a user interface 10. Other components might be present but are not explicitly referred to. In the following, "components 22" refers to the entity of components present in the housing 21.

The pressure unit 6 generally consists of a pump for generating a positive pressure or negative pressure, also called vacuum, in the expression kit 2 and a motor for driving the pump. The pressure unit 6 is connected to the housing 21 by way of a suspension 17 as will be described later in more detail. In the control unit 8 at least a processor 11 is provided for controlling the operation of the pressure unit 6. Furthermore, the control unit 8 may comprise for example a storage 12 which can be used to store operational parameters and measured values recorded in connection with the operation of the breast pump 1. The user interface 10 may comprise for example a display 13, a speaker 14, a vibrational unit 15 and one or more actuation elements 16. The components of the user interface 10 can be used to control the function and the operation of the breast pump 1 for example by way of controlling the pressure via the power of the pump and to give feedback to the user of the breast pump 1 for example by vibrational signals or by audio messages emitted by the speaker 14. Likewise, on the display 13 information about the operational state of the breast pump 1 or about the expressed milk volume in the container 5 can be displayed.

A general problem of such breast pumps 1 is the generation of noise and vibration when in use. The emissions might be annoying for the user. The main source for the emissions is the pressure unit 6 with the pump and the driving motor whose noise and vibrations are transferred to the housing 21 via mechanical coupling of the components. The emission is then transferred to the housing by way of structure-borne sound. High frequencies normally will be filtered out by noise-reducing features as described later on with reference to Fig. 2A. However, mid-range and low frequencies still pass to the housing 21 and will be perceived by the user.

Turning now to Fig. 2A, a drive unit 20 for a breast pump 1 according to the state of the art is briefly described. To eliminate noise and vibrations emitted by the pressure unit 6, it is known to arrange the pressure unit 6 on a suspension 17 which decouples the noise-generating components from the housing 21. By way of this, the higher frequencies above the so-called natural frequency will be filtered out. The natural frequency is defined by

$$\omega_e = \sqrt{\frac{c}{m}}$$

wherein c is the stiffness of the suspension 17 and m is the mass of the suspended components. While high frequencies above the natural frequency will be filtered out, all low and mid-range frequencies will be passed to the housing 21.

In Fig. 2B an embodiment of the invention is shown in a similar illustration as in Fig. 2A.

In contrast to the drive unit 20 according to the state of the art, the drive unit 20 in Fig. 2B is designed to eliminate not only high frequencies but also mid-range frequencies of the noise-generating components. To achieve this, not only the pressure unit 6, but also the control unit 8 and the user interface 10 and possible other components present in the housing 21 are arranged on the suspension 17. By way of this, the total mass of the decoupled components 22 is higher as in Fig. 2A. The natural frequency now is:

$$\omega_e = \sqrt{\frac{c}{M}}$$

with c being the stiffness of the suspension 17 and M being the mass of the suspended components 22. Comparing the two formulas it is apparent that if M is larger than m, not only high frequencies but also mid-range frequencies will be filtered out. Only low-frequency noise will now be passed to the housing 21. Low frequency noise and vibrations normally are not perceived as disturbing. Thus, the convenience of the user of the breast pump 1 can be improved.

The suspension 17 can be designed in different ways to meet the demands of respective application in the breast pump 1. Normally, the suspension 17 will comprise at least one resilient element which connects the components 22 to the housing 21. The resilient element can be a spring, for example a coil spring or a plate spring, a damping element, a vibration absorber, or an elastomeric element. The damping element can be adjustable to tune the frequencies which are eliminated from the frequency spectrum. If an elastomeric element is used, the element may comprise materials like silicone, polyurethane, natural or synthetic rubber, an elastomeric injection molded polymer (TPE) or other applicable resilient materials.

It is possible to suspend the components 22 on one resilient element only, but it is also possible to use more than one resilient element for better reliability. In case that more than one resilient element is used, all elements can be of the same type or of different types. It is for example possible to combine a spring with a number of elastomeric elements. Further combinations can provide springs with damping elements.

The internal components 22 are mounted either fixedly or removably on a base structure 23. The base structure 23 can be for example a flat element on which the components 22 can be grouped together and fastened. The weight of the base structure 23 can contribute to the total mass M. A fixed connection has the advantage that vibrations cannot disintegrate the unit. On the other hand, a removable arrangement of the components 22 on the base structure 23 allows easy change of components 22 which are out of function.

Besides, the internal components 22 can be fixedly or removably connected to each other. By way of this, the unit can be stabilized and is more resistant to the vibrations of the pressure unit 6. If the components 22 are grouped and reliably fixed to each other, the base structure 23 can be omitted and the components 22 suspended directly by the suspension 17. A coupling between the components 22 can be any suitable means like gluing, welding, connecting by straps etc. Stabilizing elements can be present to avoid disintegration of the unit during handling. Handling of the drive unit 20 will most probably include some tilting and shaking, and an occasional drop of the breast pump 1 or its components could also occur. Stabilizing elements can be for example further resilient elements which thus administrate a double function of decoupling and stabilizing.

The expression kit 2 of the breast pump 1 is connected to the drive unit 20. The connection can be permanent or removable. Due to the fact that the vibrations of the pressure unit 6 can proceed to the expression kit 2 via the pipe 7, the expression kit 2 can be connected to the housing 21 of the drive unit 20 by way of a resilient flange. The flange then can be connected to the pressure unit 6, thus decoupling the expression kit 2 from the vibrations and noise of the pressure unit 6. If the pipe 7 is resilient itself, the expression kit 2 can also be coupled directly to the pressure unit 6, as schematically shown in Fig. 2A and 2B.

The breast pump 1 can be operated by connection to an external power supply system by way of an electrical line 24. Care should then be taken not to couple the internal components 22 by way of the electrical line 24 to the housing 21. This could be prevented by a duct in the housing 21. Alternatively, the breast pump 1 can be operated by batteries.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Drive unit (20) for a breast pump, comprising

    - a housing (21),
    - internal components (22) arranged in the housing (21), and

    - a suspension (17), wherein the internal components (22) are mechanically coupled to each other to form a unit which is mechanically coupled to the housing (21) by way of the suspension (17), and
    wherein the suspension (17) comprises at least one resilient element mechanically coupling the unit of components (22) to the housing (21); and
    wherein the resilient element is a spring, a coil spring or a plate spring,
    wherein the internal components (22) are fixedly or removably arranged on a base structure (23), the base structure (23) being mechanically coupled to the housing (21) by way of the suspension (17), and
    **characterized in that** all internal components (22) arranged in the housing are mechanically coupled and are arranged on the suspension (17) via the base structure (23).

2. Drive unit (20) according to claim 1, wherein the internal components (22) comprise at least a pressure unit (6), the pressure unit (6) comprising a pump and a motor for driving the pump, a control unit (8), a user interface (10).

3. Drive unit (20) according to claim 1, wherein the resilient element includes a damping element or an elastomeric element.

4. Drive unit (20) according to claim 3, wherein the damping element is adjustable.

5. Drive unit (20) according to one of claims 1 to 4, wherein two or more resilient elements are provided, the resilient elements being of the same or of different types.

6. Drive unit (20) according to claim 5, wherein the two or more resilient elements are arranged to stabilize the internal components (22) in the housing (21).

7. Drive unit (20) according to claim 3, wherein the elastomeric element comprises silicone, polyurethane, natural or synthetic rubber, an elastomeric injection molded polymer or a combination thereof.

8. Drive unit (20) according to claim 1, wherein the internal components (22) are fixedly or removably coupled to each other.

9. Breast pump (1) comprising a drive unit (20) according to one of the preceding claims, and an expression kit (2) for positioning on a user's breast to express breast milk therefrom,
    wherein the expression kit (2) comprises an expression kit housing (4), a funnel (3), a container (5) and a pipe (7) which is connected to the drive unit (20).

**10.** Breast pump (1) according to claim 9, , wherein the expression kit (2) is fixedly or removably connected to the pressure unit (6).

**11.** Breast pump (1) according to claim 9 when being dependent on claim 2, , wherein the expression kit (2) is fixedly or removably connected to the housing (21) of drive unit (20) by way of a resilient flange with the flange being connected to the pressure unit (6).

**Patentansprüche**

**1.** Antriebseinheit (20) für eine Milchpumpe, die Folgendes umfasst:

- ein Gehäuse (21),
- im Gehäuse (21) angeordnete interne Komponenten (22), und
- eine Aufhängung (17), wobei die internen Komponenten (22) mechanisch miteinander verbunden sind und eine Einheit bilden, die über die Aufhängung (17) mechanisch mit dem Gehäuse (21) verbunden ist, und
wobei die Aufhängung (17) mindestens ein federndes Element umfasst, das die Komponenteneinheit (22) mechanisch mit dem Gehäuse (21) verbindet; und
wobei es sich beim federnden Element um eine Feder, eine Schraubenfeder oder eine Tellerfeder handelt, wobei die internen Komponenten (22) fest oder abnehmbar auf einer Sockelstruktur (23) angeordnet sind, wobei die Sockelstruktur (23) über die Aufhängung (17) mechanisch mit dem Gehäuse (21) verbunden ist, und **dadurch gekennzeichnet, dass** alle im Gehäuse angeordneten internen Komponenten (22) mechanisch verbunden sind und über die Sockelstruktur (23) an der Aufhängung (17) angeordnet sind.

**2.** Antriebseinheit (20) gemäß Anspruch 1, wobei die internen Komponenten (22) mindestens eine Druckeinheit (6) umfassen, und wobei die Druckeinheit (6) eine Pumpe und einen Motor zum Antreiben der Pumpe, eine Steuerungseinheit (8) und eine Benutzerschnittstelle (10) umfasst.

**3.** Antriebseinheit (20) gemäß Anspruch 1, wobei das federnde Element ein Dämpfungselement oder ein elastomeres Element umfasst.

**4.** Antriebseinheit (20) gemäß Anspruch 3, wobei das Dämpfungselement eingestellt werden kann.

**5.** Antriebseinheit (20) gemäß einem der Ansprüche 1 bis 4, wobei mindestens zwei federnde Elemente vorhanden sind, und wobei es sich bei den federnden Elementen um einen identischen oder um unterschiedliche Typen handelt.

**6.** Antriebseinheit (20) gemäß Anspruch 5, wobei die mindestens zwei federnden Elemente die internen Komponenten (22) im Gehäuse (21) stabilisieren.

**7.** Antriebseinheit (20) gemäß Anspruch 3, wobei das elastomere Element Silikon, Polyurethan, natürlichen oder synthetischen Kautschuk, ein elastomeres spritzgegossenes Polymer oder eine Kombination hiervon umfasst.

**8.** Antriebseinheit (20) gemäß Anspruch 1, wobei die internen Komponenten (22) fest oder trennbar miteinander verbunden sind.

**9.** Milchpumpe (1) mit einer Antriebseinheit (20) gemäß einem der vorherigen Ansprüche und einem Expressionskit (2) zum Anlegen an die Brust einer Benutzerin, um Muttermilch zu abzupumpen,
wobei das Expressionskit (2) ein Expressionskit-Gehäuse (4), einen Trichter (3), einen Behälter (5) und einen Schlauch (7) umfasst, der mit der Antriebseinheit (20) verbunden ist.

**10.** Milchpumpe (1) gemäß Anspruch 9, wobei das Expressionskit (2) fest oder abnehmbar mit der Druckeinheit (6) verbunden ist.

**11.** Milchpumpe (1) gemäß Anspruch 9, wenn dieser abhängig von Anspruch 2 ist, wobei das Expressionskit (2) über einen elastischen Flansch fest oder abnehmbar mit dem Gehäuse (21) der Antriebseinheit (20) verbunden ist, und wobei der Flansch mit der Druckeinheit (6) verbunden ist.

**Revendications**

**1.** Unité d'entraînement (20) pour un tire-lait, comprenant :

- un boîtier (21),
- des composants internes (22) disposés dans le boîtier (21), et
- une suspension (17), dans laquelle les composants (22) internes sont couplés mécaniquement l'un à l'autre pour former une unité qui est mécaniquement couplée au boîtier (21) par la suspension (17), et
la suspension (17) comprenant au moins un élément élastique couplant mécaniquement l'unité de composants (22) au boîtier (21) ; et
l'élément élastique étant un ressort, un ressort hélicoïdal ou une coupelle de ressort,
les composants internes (22) étant disposés de façon fixe ou amovible sur une structure de base (23), la structure de base (23) étant mécaniquement couplée au boîtier (21) par la suspension (17), et

**caractérisée en ce que** tous les composants internes (22) disposés dans le boîtier sont mécaniquement couplés et sont disposés sur la suspension (17) via la structure de base (23).

2. Unité d'entraînement (20) selon la revendication 1, dans laquelle les composants internes (22) comprennent au moins une unité de pression (6), l'unité de pression (6) comprenant une pompe et un moteur pour entraîner la pompe, une unité de commande (8), une interface utilisateur (10).

3. Unité d'entraînement (20) selon la revendication 1, dans laquelle l'élément élastique comprend un élément amortisseur ou un élément élastomère.

4. Unité d'entraînement (20) selon la revendication 3, dans laquelle l'élément amortisseur est réglable.

5. Unité d'entraînement (20) selon l'une des revendications 1 à 4, dans laquelle deux ou plus de deux éléments élastiques sont prévus, les éléments élastiques étant de types identiques ou différents.

6. Unité d'entraînement (20) selon la revendication 5, dans laquelle deux ou plus de deux éléments élastiques sont disposés pour stabiliser les composants internes (22) dans le boîtier (21).

7. Unité d'entraînement (20) selon la revendication 3, dans laquelle l'élément élastomère comprend du silicone, du polyuréthanne, du caoutchouc naturel ou synthétique, un polymère élastomère moulé par injection ou une combinaison de ceux-ci.

8. Unité d'entraînement (20) selon la revendication 1, dans laquelle les composants internes (22) sont couplés de façon fixe ou amovible l'un à l'autre.

9. Tire-lait (1) comprenant une unité d'entraînement (20) selon l'une des revendications précédentes, et un kit tire-lait (2) devant être placé sur un sein d'une utilisatrice afin d'en tirer le lait, le kit tire-lait (2) comprenant un boîtier de kit tire-lait (4), un entonnoir (3), un récipient (5) et un tuyau (7) qui est connecté à l'unité d'entraînement (20).

10. Tire-lait (1) selon la revendication 9, dans lequel le kit tire-lait (2) est connecté de façon fixe ou amovible à l'unité de pression (6).

11. Tire-lait (1) selon la revendication 9 lorsqu'il dépend de la revendication 2, dans lequel le kit tire-lait (2) est connecté de façon fixe ou amovible au boîtier (21) de l'unité d'entraînement (20) par une bride élastique avec la bride étant connectée à l'unité de pression (6).

FIG.1

FIG.2A

FIG.2B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050168046 A1
- EP 2196229 A1
- EP 2105151 A1
- CN 203763554 U
- US 2001038799 A1
- US 20120116299 A1